# EUROPEAN PATENT SPECIFICATION

(11) **EP 0 552 151 B1**
(45) Date of publication and mention of the grant of the patent: **05.03.1997**
(21) Application number: 91907633.1
(22) Date of filing: 02.04.1991
(51) Int. Cl.: A61K 31/74, A01N 25/10, A61K 31/14, A61K 47/48, C08K 5/19, C08L 23/04

(54) **ANTIMICROBIAL HYDROGELS**
ANTIMIKROBIELLE HYDROGELE
HYDROGELS ANTIMICROBIENS

(30) Priority: 12.10.1990 US 596268
(43) Date of publication of application: 28.07.1993
(73) Proprietor: THE DOW CHEMICAL COMPANY, Freeport, TX 77541 (US)
(72) Inventor: VAUGHN, Walter, L., Lake Jackson, TX 77566 (US); McKEAND, Thomas, J., Jr., Clute, TX 77531 (US); PATTON, Robert, T., Lake Jackson, TX 77566 (US); FINLAYSON, Malcolm, F., Houston, TX 77008 (US)
(74) Representative: Burford, Anthony Frederick
(86) International application number: US9102300
(87) International publication number: WO9206694

(56) References cited:
- EP-A- 0 341 951
- WO-A-84/00908
- GB-A- 2 010 851
- US-A- 3 325 402
- US-A- 3 872 128
- US-A- 4 111 922
- US-A- 4 766 036
- US-A- 4 766 036
- US-A- 4 920 158
- BLOCK, S.S., (1983). "Dissinfection Sterlization and Preservation", Lea & Febiger, pages 309-317.

## Description

This invention pertains to antimicrobial thermoplastic hydrogels having various forms and shapes, such as particles, fibers, and film, depending upon the desired utility. These antimicrobial hydrogels are water-swollen and water-insoluble, and have an essentially non-leachable antimicrobial compound selected from quaternary ammonium salts, protonated tertiary ammonium salts of Formula II herein, and chlorhexidine ionically bonded to a thermoplastic and are particularly useful in destroying micro-organisms. Cationic antimicrobial compounds such as quaternary ammonium salts are particularly effective. The antimicrobial hydrogels are stable to a variety of fluid media.

Antimicrobials can be either bacteriostatic or bactericidal and sometimes both. Historically, quaternary ammonium compounds have shown utility as antimicrobials. Some quaternary ammonium compounds are included as additives in various commercial formulations, e.g., cetyl pyridinium chloride is used as an additive in CEPACOL® mouthwash by Lakeside Pharmaceuticals. Other commercial antimicrobial compounds are effective as additives for aqueous systems for disinfecting articles. One such antimicrobial useful in this manner is didecyl dimethyl ammonium chloride, trademarked BARDAC®225O and made by Lonza, Inc. Even though these antimicrobials have proven effective, their utility is concentration dependant and, therefore, is diminished by dilution.

In the article by Ackart et al. entitled "Antimicrobial Polymers", J. Biomed. Mater. Res., Vol. 9, pages 55-68 (1975), antimicrobial ionomers prepared from ethylene/acrylic acid interpolymers and alkylbenzyldimethylammonium cations, particularly 8-hydroxyquinolinium, are disclosed. According to Ackart et al., "these ionomers would not be expected to provide significant protection to the user from pathogenic bacteria on the surface of an article fabricated from the ionomers" and hence would be classified as bacteriostatic. The mechanism of operation of the antimicrobials of Ackart et al. is stated to be such that a liquid transfer medium is needed in order for the ionomers to be effective in environments in which no direct liquid-liquid or solid-liquid interphase transfer occurs.

US-A-3,872,178 discloses antimicrobial ammonium salts of carboxyl-containing α-olefin polymers. The ammonium cations of said salts are derived from certain N-unsubstituted pyridine or quinoline derivatives. A preferred ammonium cation is 8-hydroxyquinolinium.

US-A-2,951,766 (White) discloses antiseptic plastics which, upon contact with moisture or water, release the antiseptic from the material. The antiseptic is said to be dissolved or dispersed in a hydrophilic plastic material, but not chemically reacted with the plastic material. The plastic materials useful for this invention are water or alcohol-water soluble materials unless cross-linking agents are added. The antiseptic plastics disclosed in White are bactericidal, but the antiseptic or antimicrobial chosen is released from the plastic upon contact with water or moisture. Release of antiseptic may not be environmentally proper, depending upon the application, and therefore continues to be a commercial need.

Hydrogels have been known to be useful for entrapping or immobilizing biologically active molecules such as antibiotics and anti-bacteria agents. Hydrogels for Medical and Related Applications, (ACS Symposium Series 31 (1976)), by Joseph D. Andrade, defines hydrogels as "three dimensional networks of hydrophillic polymers, generally covalently or ionically crosslinked, which interact with aqueous solutions by swelling to some equilibrium value." A hydrogel is further defined as exhibiting "the ability to swell in water and retain a significant fraction (e.g., >20 percent) of water within its structure, but which will not dissolve in water." The hydrogels are stated to be prepared by solution polymerization of a monomer in the presence of a cross-linking agent and then entrapping the active biomolecule within the network structure which forms or by covalently immobilizing active molecules to hydrogels surfaces, such as coupling biomolecules to gels containing carboxyl groups.

Water-swollen, but water-insoluble hydrogels characterized as having pendant alkali metal carboxylate groups or ammonium carboxylate groups reacted with at least one antimicrobial compound selected from quaternary ammonium salts, protonated tertiary ammonium salts of Formula II herein, and chlorhexidine thereby ionically bonding the antimicrobial compound to the interpolymer have now been discovered to form antimicrobial hydrogels. The antimicrobial hydrogels are surprisingly useful in many forms, e.g., dispersions, fibers, films and particles. The antimicrobial hydrogels of the present invention have an essentially non-leachable antimicrobial ionically bonded to a thermoplastic interpolymer. The antimicrobial hydrogels of the present invention are effective antimicrobials, killing many micro-organisms on contact. The antimicrobial hydrogels of the present invention are surprisingly stable and also regenerable. The antimicrobial hydrogels of the present invention are useful in many areas, including stabilizing wood pulp and wood chips against souring, destroying microorganisms in fluids such as milk, juice and water, preventing cosmetic products from becoming contaminated by microbes, as additives in foams, and in forming antimicrobial paper.

One aspect of the invention is a method of producing a water-swollen, but water-insoluble antimicrobial hydrogel comprising at least one vinyl addition thermoplastic interpolymer having pendant alkali metal carboxylate groups or other salts thereof in various forms, such as fiber, film or particles, and at least one essentially non-leachable antimicrobial compound selected from quaternary ammonium salts, protonated tertiary ammonium salts of Formula II herein, and chlorhexidine ionically bonded to substantially all of the pendant carboxylate groups. The thermoplastic interpolymer can be either linear or branched, or a mixture of these.

The thermoplastic interpolymer can be a reacted or grafted mixture of ethylene-alpha olefins and carboxylic acids or anhydrides (e.g., ethylene-methacrylic acid interpolymer) but is preferably an ethylene-acrylic acid interpolymer (e.g., the homogeneous, random interpolymers of ethylene with alpha-olefinically unsaturated carboxylic acids or esters made in accordance with US-A-4,599,392, or the homogeneous, random interpolymers of ethylene with alpha-olefinically unsaturated carboxylic acids or esters made with telogenic modifiers in accordance with US-A-4,988,781). The thermoplastic interpolymer can be in any shape, but is preferably formed into a shape dependant primarily upon the final utility, such as fiber or film. The thermoplastic interpolymer can also be in the form of particles and used in a dispersion.

The antimicrobial compounds useful in the present invention are effective antimicrobials which must be essentially non-leachable when reacted with the vinyl addition thermoplastic interpolymer and subsequently contacted with deionized water. Chlorhexidine is a particularly effective antimicrobial for use in the invention. Quaternary ammonium antimicrobial salts are also particularly useful in forming the antimicrobial hydrogels of the present invention and can be any member of a known class of quaternary ammonium antimicrobial compounds. Preferably the antimicrobial is one which has a molecular structure corresponding to one of the following formulae: 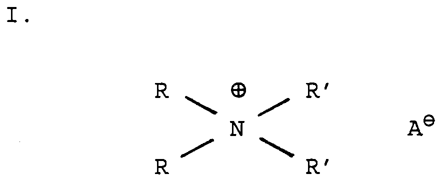 wherein
each R' independently is -CH₃ or -C₂H₅ and each R independently is C₂H₅-C₂₀H₄₁
A^{⊖} = compatible neutralizing inert anion, 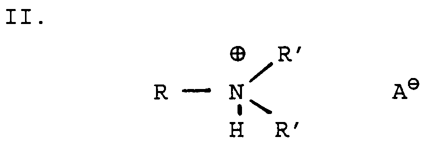 wherein
each R' is independently -CH₃ or -C₂H₅ and R = C₂H₅-C₂₀H₄₁
A^{⊖} = compatible neutralizing inert anion,
or 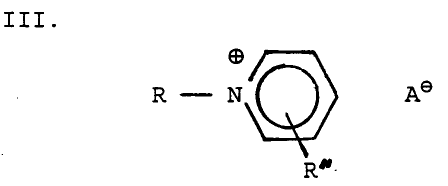 wherein
each R = C₂H₅-C₂₀H₄₁
R'' = inert substituent or -H
A^{⊖} = compatible neutralizing inert anion.

In each structure, the "R" group can also be a ring structure, such as a phenyl group. Didecyl dimethyl ammonium chloride and didecyl dimethyl ammonium bromide (generically described by Formula I), dimethyl benzyl ammonium chloride and dimethyl benzyl ammonium bromide (generically described by Formula II), and cetyl pyridinium chloride and cetyl pyridinium bromide (generically described by Formula III), or mixtures thereof are most preferred as the antimicrobials for practising the present invention.

The antimicrobial hydrogel can be formed from the thermoplastic interpolymer by a two step method or, preferably a-one step method. The two step method comprises reacting at least one interpolymer having pendant carboxylate groups, preferably at least one ethylene-acrylic acid (EAA) interpolymer, in the appropriate shape with an aqueous pH basic solution (e.g., sodium hydroxide, potassium hydroxide, ammonium hydroxide) for a time and at a temperature sufficient to enable reaction between the base and the acrylic acid functionality to form a cation acrylate, (e.g., sodium acrylate, potassium acrylate, or ammonium acrylate). The extent of the reaction is dependant upon many factors, such as the alkaline concentration of the pH basic solution, the temperature of the solution, the time of contact between the interpolymer and the solution, the percent acid functionality present in the EAA interpolymer and the form or size of the EAA interpolymer. The second step in the two step method comprises contacting the cation acrylate with an aqueous antimicrobial solution comprising at least one quaternary ammonium salt having antimicrobial properties for a time sufficient to enable the antimicrobial salt to ionically bond to the cation acrylate site, thereby discharging the cation and forming the antimicrobial hydrogel.

Alternately, a one step process can be used to form the antimicrobial hydrogel. The one step process comprises simultaneously contacting the shaped thermoplastic interpolymer with the quaternary ammonium antimicrobial salt in an aqueous pH basic solution. The pH basic solution can have a pH of at least pH 7.1. Preferably the thermoplastic interpolymer comprises at least one EAA interpolymer. The extent of the conversion from the acid form of the EAA interpolymer to the antimicrobial hydrogel form is most preferably essentially complete. That is, substantially all (i.e., at least 75-80 percent) of the pendant acrylic acid groups are ionically bonded with the antimicrobially active salt. The antimicrobial hydrogel produced by either the two step method or the one step method in the form of a particle can be useful as a dispersion or as an additive in, for example, toothpaste, in hand cream, and mouthwash. The antimicrobial hydrogel produced by either the two step method or the one step method in the form of a fiber can be useful in controlling micro-organisms in various fluids, including water (e.g., commode water), high purity chemicals, and air or it can be useful in the form of bonded fibers in a nonwoven material. The hydrogels can also reduce micro-organism levels in recirculating white water used in processing wood pulp into paper.

The antimicrobial hydrogels of the present invention are fully hydratable, i.e., the hydrogels can be either substantially dehydrated (e.g., to a moisture content as low as 10-14 percent), partially hydrated or fully hydrated.

In another aspect, the hydrogels of the present invention are useful as a means of absorbing aqueous fluids. The hydrogels are capable of absorbing up to 50 percent of their weight in water. The amount of water absorbed in the hydrogels is dependant upon many variables, including the amount of reactive pendant alkali metal or ammonium carboxylate groups present in the base polymer, the degree of orientation of the base polymer and the antimicrobial chosen for forming the hydrogel.

In still another aspect, the invention is a stable antimicrobial hydrogel capable of withstanding exposure to oils, fats and solvents. The hydrogels are stable to oxidative effects and chemical contact. Generally, the antimicrobial is retained within the hydrogel after 7 days of continuous deionized water washings in amounts of at least 90 weight percent, preferably at least 94 weight percent.

Another aspect of the invention is that the antimicrobial hydrogel can be optionally crosslinked to form a hydrogel having a higher melting point. A higher melting point antimicrobial hydrogel has use, for example, in high temperature fluid treatment.

The melting point of the hydrogel of the present invention can optionally be elevated by ion exchanging at least one mono-, di-, and/or tri-valent metal cation with the pendant alkali metal carboxylate groups. The Fisher-Johns melting point of the hydrogel of the present invention can be elevated from 66°C to as high as 141°C. For example, a hydrogel made in accordance with the present invention having a melting point of 66°C, has a melting point of 141°C when reacted with ferric sulfate such that the metal reacted hydrogel has 1900 ppm ferric ion (Fe+++).

### Hydrogel Preparation

Preparation of the antimicrobial hydrogel of the present invention can be accomplished by various techniques, three of which are described below:

### Starting material

The base polymer is a vinyl addition interpolymer, preferably shaped in a form suitable for a specific end use application. For example, the base polymer can be an ethylene-methacrylic acid interpolymer (EMAA) or an ethylene-acrylic acid interpolymer (EAA) or the base polymer can be a blend of EAA interpolymers, or blends of EAA interpolymers with other thermoplastic polymers, (e.g., polypropylene, linear low density polyethylene, low density polyethylene, high density polyethylene or a similar polyolefin). The base polymer preferably has at least one EAA interpolymer as one component. The base polymer or polymer blend is then formed into a particular shape, dependant upon the final hydrogel application area. For example, if the hydrogel were to be used in contact with a fluid, such as water, milk, assorted juices or air, to kill or control micro-organisms contained therein, then the hydrogel is advantageously in the form of a fiber. The fiber can then be wound or configured into a cartridge for the actual contacting step. In this particular instance, the base polymer is preferably first melt spun into a fiber. The fiber can be of any appropriate diameter, such as strands, monofilaments, or fine denier, useful, for example, in dental floss, and may be a variety of shapes, merely adjusted by utilizing a die having the orifice shaped differently, such as circular shape, a star shape or trilobal shape. The fiber can also be in the form of a bicomponent fiber, e.g. a sheath/core configuration or a side-by-side configuration. In the case of a sheath/core configuration, the base polymer to be converted into a hydrogel can be either the sheath resin or the core resin or two different base resins can be utilized with one being the sheath and one being the core. Preferably, the sheath resin is the base resin. Those skilled in the art of fiber formation from thermoplastic materials know the various types of spinning which render the chosen polymer into a fiber form, such as continuous filament spinning, staple fiber formation, hollow fiber manufacture, spunbonded fabric manufacture including multilayer or entangled structures (i.e., one layer may be EAA interpolymer and another layer may be a lower melting fiber forming material, such that the lower melting material forms bonding sites when the entire structure is passed through thermal calenders) and melt blown fiber or fabric manufacture including combinations of spunbond/melt blown/spunbond structures with the present invention residing in any or all of the sections, and any of the other known fiber forming methods from thermoplastic interpolymers are suitable for the practice of this invention. Additionally, the fiber forming step can impart a secondary or tertiary orientation to the fiber, surprisingly enhancing some of the properties of the hydrogel formed from that fiber, such as the ability of the hydrogel to increase the capacity for holding water. Such secondary orientations are commonly employed in the formation of staple fiber wherein godets are operated at different speeds such that the fiber is stretched or drawn between the godets and the fiber diameter is consequently reduced and the resultant fiber is more highly oriented and stronger. Use of higher orientation in the fiber forming step in the present invention affects the hydrogel by not only increasing the strength of the starting interpolymer in fiber form, but also increasing the amount of water which the hydrogel may contain. The fibers or filaments formed from the base polymer can then be placed in the form of a cartridge for ultimate use in a filter system, such as that used for filtering water or alternately can be converted directly into the hydrogel form by one of the two methods described in detail further in this disclosure.

The base polymer can alternately be formed into fine diameter particles. These particles can be formed, for example, by grinding the polymer and then sieving to narrow the particle size distribution. The particle size can be a range of sizes, or it can be a specific size distribution, depending upon the desired utility. The particle size can be colloidal (e.g., useful in forming dispersions) or it can be as large as pellets. By varying the size of the particles of the base polymer, the hydrogel particles formed from them can be useful in various products, such as toothpaste, mouth wash, soap, shampoo, ointment and hand cream. Fine diameter hydrogel particles can be added to wood pulp slurries either as solids, or formed in-situ as particles in the pulp, to stabilize against souring (i.e., micro-organism infestations). The particle size of the base polymer and ultimately the hydrogel will influence the ability of the hydrogel to remain in suspension in the fluid in which it is contained and thus is chosen accordingly. Conversely, the hydrogel can be made using the process disclosed herein, and then ground to the desired particle size for use in a dispersion. Hydrogel particles can also be added to a foam or a foam structure to impart biocidal or antimicrobial characteristics. The foam, for example, can comprise a polyolefin, polystyrene, latex, urea, isocyanurate, polyurethane or a cellulosic polymer.

The base polymer can also be in the form of a film or a film laminate or a coating. Film can be formed by any of the techniques known to those skilled in the art of film formation, but these are commonly known as cast film, blown film and extrusion coating operations. In the case of an extrusion coating operation, the base polymer is extrusion coated onto another substrate, e.g, a nylon film. Ethylene-acrylic acid interpolymers are particularly well known for their ability to adhere to polar substrates and consequently, if ethylene-acrylic acid interpolymer were used as the extrusion coating resin onto a nylon substrate, then a strongly adhered laminate would be formed. Alternately, the base polymer can be in the form of a coating on a substrate, such as that obtained by coating the base polymer onto bristles, e.g., common nylon toothbrush bristles. The resultant conversion of this film form or coating of the base polymer (ethylene-acrylic acid interpolymer in this particular example) into the hydrogel form would then be accomplished by one of the techniques described further in this disclosure.

Alternately, the antimicrobial hydrogel can be formed and subsequently thermally extruded into a desired shape. The formed hydrogels can also be blended with other thermoplastics and melt processed into another desired shape, such as fiber, film, particles or pellets. Thermoplastics suitable for blending with the hydrogels of the present invention include, but are not limited to polyolefins such as polypropylene, low density polyethylene or LDPE, linear low density polyethylene or LLDPE, other base polymers such as ethylene-acrylic acid interpolymers, and other vinyl addition interpolymers having pendant alkali metal or ammonium carboxylate groups. The antimicrobial hydrogels of the present invention are thus thermally stable and can be reprocessed into a variety of shapes, including fiber, film and other molded articles, either by themselves or in blends with other thermoplastics.

### Method One (The One Step Process):

The first method of preparing the hydrogel utilizes a one step method. For the examples contained and described in this disclosure unless stated otherwise, the base polymer is ethylene-acrylic acid (EAA) interpolymer having a melt index (which is inversely proportional to molecular weight) of 300 grams/10 minutes measured according to ASTM D-1238 (E) (190°C/2.16 kg), an acrylic acid content of 20 percent by weight of the interpolymer and a Fisher-Johns melting point of 96°C. Other base polymers can be employed which are also EAA, and these can have different percents acrylic acid and/or different molecular weights. The base polymer molecular weight will be dictated by the end use and form in which the hydrogel is desired. For example, in the case of film manufacture, the base polymer can have a melt index of between 0.1 and 30 grams/10 minutes. If the base polymer were to be made into particles, the melt index can vary from 0.1 to more than 3000 grams/10 minutes. If the base polymer were to be made into fiber, then the melt index is desirably between 20 and 2600 grams/10 minutes.

The first method of forming the hydrogel from the base interpolymer is by treating the EAA interpolymer in one step using a pH basic aqueous antimicrobial solution. By adjusting and maintaining the pH of the aqueous antimicrobial solution to a basic pH of at least pH 7.1 using sodium hydroxide, the reaction between the sodium ions forming the sodium acrylate ionomer and the antimicrobial quaternary ammonium salt ion most preferably proceeds essentially to completion if desired. That is, essentially all (i.e., at least 80 percent) of the acrylic acid moieties are reacted with the sodium hydroxide to form pendant alkali metal carboxylate groups and subsequently also reacted with the quaternary ammonium compound in situ. The time needed for this reaction in a basic medium is minimized by continually supplying sodium ions to the acrylic acid sites while simultaneously exchanging the quaternary ammonium salt with the sodium ions previously attached to the acrylic acid sites thereby driving the equilibrium towards the hydrogel formation and the reaction essentially to completion. In this manner, the quaternary ammonium salt is ionically bonded to the pendant alkali metal carboxylate groups. The one step process will not proceed to the same extent when using an acid medium and is therefore not as desirable for use in the present invention.

The conversion yield of sodium acrylate ionomer to quaternary ammonium ion when using the one step process described in a basic medium is desirably at least 30 percent, preferably at least 60 percent, and most preferably at least 80 percent.

### Method Two (The Two Step Process):

Step 1: The base polymer configured into the desired shape is treated by swelling in a pH basic solution (e.g., sodium hydroxide). The temperature of the caustic solution may be in a broad range, but it is preferably above room temperature, i.e., greater than 25°C, and below the melting point of the EAA. The caustic solution, e.g., sodium hydroxide (NaOH) or potassium hydroxide (KOH), can be at different strengths or concentrations. Generally the higher the concentration, the faster the reaction of the caustic with the acrylic acid groups of the EAA. The pH of the NaOH solution is preferably at least pH 7.1. Swelling the EAA interpolymer in caustic solution can take various lengths of time depending upon the shape of the EAA and the degree of reaction desired with the acid groups of the EAA. Generally, at least 30 percent by weight of the acrylic acid groups in the EAA are reacted with the NaOH, forming an ionomer having 3.5 percent by weight sodium ionically bonded to the EAA to form an ethylene-sodium acrylate ionomer having a melt index of 0.1 grams/10 minutes and a melting point of 250-252°C, as determined by a Fisher-Johns melting point apparatus. Most preferably, when the reactions are complete, at least 80 percent by weight of the acrylic acid groups in the EAA are reacted with NaOH. Excess caustic solution is washed off the ionomer with deionized water.

Step 2: The ethylene-sodium acrylate ionomer is then contacted and reacted with an antimicrobial in a dilute aqueous solution having a pH of 6-7 at a concentration at least one mole of quaternary ammonium compound to one mole of acrylic acid, with the antimicrobial preferably being a quaternary ammonium salt compound. Preferably, at least one antimicrobial is chosen having a chemical formula from the group described earlier in this disclosure.

The aqueous antimicrobial solution is usually pumped around and through the ionomer to ensure good contact for a time sufficient to exchange the sodium groups of the ethylene-sodium acrylate ionomer with the quaternary antimicrobial compound to form an antimicrobial hydrogel having a melt index of 100 grams/10 minutes and a Fisher-Johns melting point ranging from 48°C to 88°C. Generally, the greater the extent of the reaction, the higher the percent nitrogen in the hydrogel and the lower the melting point. The contact time for the aqueous antimicrobial solution is dependant upon the extent of reaction desired, but it is preferably at least 1 hour and as long as 24 hours. For the examples disclosed herein, the antimicrobial compound used is didecyl dimethyl ammonium chloride unless stated otherwise. Preferably at least 80 percent of the sodium ions are exchanged with the quaternary compound and most preferably at least 99 percent of the sodium ions are reacted. An ethylene-sodium acrylate ionomer having 4.2 percent by weight sodium content will have 20 parts per million (ppm) sodium content after reaction with dimethyldidecyl ammonium chloride. The nitrogen content on the dry or dessicated hydrogel sample is 1 percent by weight, which is equivalent to 23 percent by weight of the quaternary ammonium salt.

### Method Three

A third method of making the antimicrobial hydrogel from ethylene-acrylic acid interpolymers uses melt mixing of the components. The hydrogel is prepared by reacting molten ethylene-acrylic acid interpolymers with the antimicrobial, e.g., didecyl dimethyl ammonium chloride, while either simultaneously adding a caustic solution, or by incrementally adding caustic solution. This process advantageously increases the molecular weight of the antimicrobial hydrogel formed while also enabling the reaction to the antimicrobial hydrogel stage to be performed using one step. The reaction is carried out in any melting pot, preferably an extruder or a Brabender mixer.

### EXAMPLE 1

An ethylene-acrylic acid interpolymer having a melt index of 300 grams/10 minutes and 20 percent acrylic acid was converted into the appropriate form by melt extruding into strands and converting into antimicrobial hydrogels. Using the two step method described in Method Two above, 100 grams of the strand was placed into a continuously stirred beaker containing an excess of 0.5 Normal NaOH (or 2 weight percent) solution held at 55°C. The mixture was digested for 5 hours to convert the strands into an ethylene-sodium acrylate ionomer. The ethylene-sodium acrylate ionomer was then washed repeatedly with deionized water to remove excess caustic. The form of the strands was in a swollen state such that the diameter was greater than it was at the start of the experiment. The swollen strands were then hammer-milled into fine diameter short fibrils and screened into 20-40 mesh (850-400 µm) size fibrils. The fibrils had a sodium content of 3.5-4.5 percent by weight at this stage of the treatment.

After the excess caustic was washed from the swollen sodium acrylate ionomer fibrils, the sample of fibrils was contacted with 500 milliliters (mls) of a 5 percent by weight aqueous solution of didecyl dimethyl ammonium chloride by placing the fibrils in a 5 cm (2 inch) diameter by 50 cm (20 inch) glass column which was fitted with spun glass fiber in the column outlet to prevent the sodium acrylate ionomer from plugging it. The swollen fibrils filled about half of the glass column. The didecyl dimethyl ammonium chloride solution was recirculated through the fibrils at a flow rate of 12-20 bed volumes per hour, or 6-10 liters/hour, and at a total treatment time of 18 hours forming antimicrobial hydrogel fibrils. At this stage, the diluted didecyl dimethyl ammonium chloride solution was diverted from the column and the column purged with 20 liters of deionized water. The fibrils were removed after purging with water and air dried. The water-swollen antimicrobial hydrogel fibrils were soft and rubbery at this point. The fibrils had a sodium content of 75 ppm and a nitrogen content of 1.7 percent based on dry fiber weight, which corresponds to 40.1 percent by weight of the quaternary ammonium compound. The conversion reaction of the sodium acrylate ionomer to the antimicrobial hydrogel state was 80.9 percent.

Effectiveness of the hydrogel fibrils was demonstrated by testing the hydrogel samples using the EASICULT® TTC test kit designed by ORION DIAGNOSTICA. The EASICULT® TTC test kit measures Colony Forming Units of micro-organisms per milliliter of test solution (CFU/ml). Colony forming units were visually evident on the test slides as red colonies or "dots". The tested slides were visually compared against photographs of standard slides to obtain CFU/ml of the test fluid in question. Test fluid was prepared by using 3.8 liters (1 US gallon) of actual pond water diluted with 34 liters (9 US gallons) of deionized water. Nutrient broth (manufactured by DIFCO Laboratories) was added to the test fluid in 0.5 gram portions to stimulate micro-organism growth.

To determine antimicrobial efficacy, approximately 70 grams of the water-swollen hydrogel fibril sample (30.3 grams of fibril on a dry weight basis) was placed in a 100 ml glass burette to a depth of 100 mm. Test fluid containing approximately 1,000,000 CFU/ml of micro-organisms was pumped through the antimicrobial hydrogel packed burette at 20, 40, 60 and 120 bed volumes per hour, corresponding to contact times of 3, 1.5, 1 and 0.5 minutes, respectively.

The test fluid effluent was collected and tested for micro-organism content by dipping a fresh EASICULT® slide into the test fluid. Each dipped slide was placed into an incubator oven at 34°C (93°F) and incubated for 48 hours. After the incubation period, each of the dipped, incubated slides was removed from the incubator oven and visually compared against the standard test slides to determine and assign the number of CFU/ml for that particular sample. For test values reported below the test limit described in the EASICULT® TTC kit (i.e., 1000 CFU/ml), the results were obtained by serial dilution of a known organism count solution and comparing test slide colony counts. Results of "<10*"* CFU/ml means that there were no colony forming units seen on the slide.

The fibrils of EAA which were converted into antimicrobial hydrogels reduced the micro-organisms content of the effluent at all contact times tested to <10 CFU/ml of micro-organisms. Thus, under the conditions of this test, the antimicrobial hydrogels demonstrated essentially complete kill (>99.999 percent) of micro-organisms in less than 30 seconds contact time.

### EXAMPLE 2

An ethylene-acrylic acid interpolymer having the properties described in Method Two above was converted into the appropriate form by extruding into continuous strands at a temperature of 113°C (235°F) to strand diameters of 3.18 mm (0.125 inches) and 7.6 mm (0.3 inches). The strands were made on a small 1.9 cm (0.75 inch) diameter extruder system having two temperature zones and were extruded under 1480 kPa (200 psig) into a bucket of water for solidification. Portions of the 3.18 mm (0.125 inch) strand were placed into a vacuum oven and annealed for 15 hours between 30°C and 50°C. Other portions of the unannealed strands were stretched and oriented by hand according to the following table:

| Sample No. | Stretch Ratio | Comments | Diameter as Stretched (mm) |
|---|---|---|---|
| 2A | NA | As Extruded | 3.18 (0.125 in) |
| 2B | NA | After Annealing | 3.18 (0.125 in) |
| 2C | 2:1 | Stretched; No Annealing | 1.6 (0.063 in) |
| 2D | 3.5:1 | Stretched; No Annealing | 0.9 (0.036 in) |
| NA = Not applicable, i.e., not stretched | | | |

Samples 2A through 2D were treated using Method One above (One step method) to form the corresponding hydrogels. The process to form these hydrogels utilized 20 grams of a 50 percent by weight solution of didecyl dimethyl ammonium chloride having 10 percent ethanol and 40 percent water and 130 grams of deionized water, with the entire solution brought to a pH of 14 using 5.7 grams of aqueous sodium hydroxide forming a basic didecyl dimethyl ammonium chloride solution. For each sample 2A through 2D, a 5 gram portion of the strand was placed in a 4 US ounce (120 cm³) glass bottle . The basic didecyl dimethyl ammonium chloride solution was then poured into the bottle and allowed to stand at room temperature (25°C) for 12-24 hours or until the reaction was complete. Heating and/or stirring the mixture will cause the reaction to proceed at a faster rate. Reaction acceleration may be accomplished, for example, by heating the mixture to 58-60°C. After contacting the basic didecyl dimethyl ammonium chloride solution, the antimicrobial hydrogel strands were thoroughly washed with deionized water and allowed to equilibrate to room temperature and humidity conditions. The following table describes the properties of the strands after conversion to the antimicrobial hydrogel form:

| Sample No. | Stretch Ratio | Weight Percent Nitrogen | Weight Percent Quat. Ammonium Compound | Weight Percent Moisture* | Percent Conversion of Acrylic Acid |
|---|---|---|---|---|---|
| 2A | NA | 1.1 | 24.97 | 29.61 | 46 |
| 2B | NA | 1.1 | 24.97 | 25.62 | 46 |
| 2C | 2:1 | 1.6 | 36.32 | 40.45 | 66 |
| 2D | 3.5:1 | 1.9 | 43.13 | 48.69 | 78 |
| NA = Not applicable, i.e., not stretched | | | | | |

| | | | | | |
|---|---|---|---|---|---|
| *Percent moisture is measured by drying in a vacuum oven at 45-50°C for 12-18 hours and measuring weight loss of the strands | | | | | |

Samples 2C and 2D show that stretching the strands before making a hydrogel, while using the one step process for forming the hydrogel, surprisingly enhances the ability of the hydrogel to increase the percent water retained and the conversion of the acrylic acid to the hydrogel form.

Effectiveness of the hydrogel formed using Method One as described above was tested using a zone of inhibition test. The zone of inhibition test demonstrates effectiveness of a antimicrobial on an agar plate which has been contaminated with a micro-organism-contaminated test fluid. The test fluid was prepared by using 3.8 liters (1 US gallon) of actual pond water diluted with 34 liters (9 US gallons) of deionized water. Nutrient broth (manufactured by DIFCO Laboratories) in 0.5 gram portions was added to the test fluid to stimulate micro-organism growth. Fresh agar plates (Plate Count Agar w/TTC (2,4,5-triphenyltetrazolium chloride) from Remel in Lenexa, Kansas) were then dipped three times into the test fluid. Each sample of hydrogel was placed onto the dipped agar plate and the agar plate containing the hydrogel placed into an oven incubator and allowed to incubate for 48 hours at 30-34°C (86-93°F). After the incubation, the agar plate containing the hydrogel was observed for inhibition of growth. The zone of inhibition was the area around the sample which does not support micro-organism growth. The zone of inhibition test measures the migratory nature of the antimicrobial in question, since the sample is stationary on the agar plate and the zone is measured about the periphery of the sample. The antimicrobial hydrogels formed by the present examples had minimal zones of inhibition, with the zone on the agar plate only beneath the sample and labeled as NGCA (No Growth on the Contact Area). The table below describes the test results:

| Sample No. | Weight Percent Nitrogen | Weight Percent Quat. Ammonium Compound | Zone of Inhibition* |
|---|---|---|---|
| 2A | 1.1 | 24.97 | TG |
| 2B | 1.1 | 24.97 | NGCA |
| 2C | 1.6 | 36.32 | NGCA |
| 2D | 1.9 | 43.13 | NGCA |

| | | | |
|---|---|---|---|
| *TG = Trace Growth under the sample NGCA = No Growth on the Contact Area | | | |

Thus, the antimicrobial hydrogels do not substantially leach the antimicrobial to the surrounding agar plate media, but still prove effective as antimicrobials which control micro-organisms on contact as evidenced by no growth on the contact area.

### EXAMPLE 3

A hydrogel was prepared using Method Three described above, wherein a portion of ethylene-acrylic acid interpolymer is added to a Brabender mixer at 125°C and allowed to melt and mix. Solid caustic (sodium hydroxide) and didecyl dimethyl ammonium chloride were added to the mixer when the EAA was melted and allowed to mix until the appearance was uniform (2 minutes at 150 RPM). The polymer mixture was then removed from the mixer and pressed into films which are cut into small pieces. The small pieces were washed overnight in deionized water before testing for antimicrobial activity. The chart below identifies quantities of starting materials as well as theoretical and actual nitrogen content of the hydrogels formed using the melt blending process:

| Sample No. | Amount of EAA Added (grams) | Amount of NaOH Added (grams) | Amount of Biocide Added (grams) | Theoretical Percent Nitrogen in the Hydrogel | Actual Percent Nitrogen in the Hydrogel |
|---|---|---|---|---|---|
| 3A | 30 | 6 | 5 | 0.46 | 0.4 |
| 3B | 30 | 6.7 | 15.1 | 1.3 | 0.7 |

Effectiveness of the melt manufactured hydrogels was demonstrated by testing the hydrogel samples using the EASICULT® TTC test kit designed by ORION DIAGNOSTICA as described in Example 1. To determine antimicrobial efficacy, each hydrogel sample was placed in 500 mls of test fluid (prepared as described in Example 1) which contained approximately 10,000 CFU/ml of micro-organisms. The level of the hydrogel in the test fluid was 500 ppm by weight of active bound quaternary ammonium compound. The test fluid containing the hydrogel sample was continuously stirred at room temperature and, at the times indicated in the table, a fresh EASICULT® slide was dipped into the test fluid. Each dipped slide was placed into an incubator oven at 34°C (93°F) and incubated for 48 hours. After the incubation period, each of the dipped, incubated slides was removed from the incubator oven and visually compared against the standard test slides to determine and assign the number of CFU/ml for that particular sample. As described in Example 1, test values reported below the test limit described in the EASICULT® TTC kit (i.e., 1000 CFU/ml) were obtained by comparing the test slide colony count with a previously prepared standard curve (prepared by serial dilution of a known organism count solution). Results of "<10*"* CFU/ml means that there were no colony forming units seen on the slide. Data for the tests using the hydrogels of the present experiment appears in the following table:

| Sample No. | Day 1 CFU/ml | Day 2 CFU/ml | Day 5 CFU/ml | Day 6 CFU/ml | Day 7 CFU/ml | Day 8 CFU/ml |
|---|---|---|---|---|---|---|
| 3A | <10 | 10 | 50,000 | 10,000 | 50,000 | 50,000 |
| 3B | <10 | <10 | <10 | <10 | 10,000 | 100,000 |

These data indicate that while both samples demonstrate antimicrobial activity, the samples with the greater extent of conversion (as represented by nitrogen content) had longer utility in use.

### EXAMPLE 4 - not an example of the invention; for comparison only.

EAA was reacted with didecyl dimethyl ammonium chloride in the melt without using an aqueous base. Thirty five grams of EAA were added to a Brabender at 100°C and allowed to melt and mix. Ten grams of didecyl dimethyl ammonium chloride were added to the molten EAA and allowed to mix until the polymer blend has a uniform appearance. The polymer blend was removed from the Brabender, pressed into film and cut into small pieces. These small pieces were then repeatedly washed with deionized water. The samples produced in this manner had a maximum theoretical nitrogen content of 1.1 percent and a measured nitrogen content of 0.8 percent, seemingly indicating a conversion of 70 percent, but were not water-swollen. Test fluid was prepared as described in Example 1 containing approximately 10,000 CFU/ml of micro-organisms (measured using the EASICULT® system). The efficacy of the polymer blend sample was determined by using the EASICULT® test procedure described in Example 3. The results are shown in the table below:

| Day 1 CFU/ml | Day 2 CFU/ml | Day 5 CFU/ml | Day 6 CFU/ml | Day 7 CFU/ml | Day 8 CFU/ml |
|---|---|---|---|---|---|
| 100,000 | 100,000 | 100,000 | 100,000 | 10,000,000 | 1,000,000-10,000,000 |

As the data indicate, the sample made without adding the base solution to the polymer melt was not effective in destroying or controlling micro-organisms and thus is not an example of the present invention.

### EXAMPLES 5-10 and Comparative Example 11

A series of antimicrobials were used to form various hydrogels using the same one step method described in Example 2 with the same starting polymer (EAA) melt spun into approximately 50 micro-meter diameter fiber, with the exception that the antimicrobial in Example 2 was substituted with each of the respective antimicrobials in the subsequent Examples. Test fluid was prepared as described in Example 1 containing approximately 10,000 CFU/ml of micro-organisms (measured using the EASICULT® system). Using the EASICULT® test procedure described in Example 3, the efficacy of each of the individual hydrogels was determined. The antimicrobials used in these examples, their chemical identity, percent nitrogen and the percent active bound quaternary ammonium compound appears in the following table:

| Antimicrobial Example | Chemical Identity | Tradename | Percent Nitrogen | Percent Active Quat. Ammon. Cmpnd. |
|---|---|---|---|---|
| 5 | DIMETHYL BENZYL AMMONIUM CHLORIDE | BARQUAT® 4280 | 1.5 | 35.97 |
| 6 | DIMETHYL DICOCO AMMONIUM CHLORIDE | ARQUAD* 2C-75 | 0.5 | 32.59 |
| 7 | CETYL DIMETHYL ETHYL AMMONIUM CHLORIDE | NA | 1.5 | 62.5 |
| 8 | CETYL TRIMETHYL AMMONIUM BROMIDE | NA | 1.5 | 58.33 |
| 9 | CETYL PYRIDINIUM CHLORIDE | NA | 1.3 | 28.28 |
| 10 | DIDECYL DIMETHYL AMMONIUM CHLORIDE | BARDAC® 2250 | 1.7 | 39.96 |
| NA = Not Available | | | | |

| | | | | |
|---|---|---|---|---|
| ® Registered trademark of LONZA, Inc. | | | | |
| *Made by AKZO Chemie America | | | | |

Comparative Example 11 measures the micro-organism increase in the test fluid without hydrogels present, i.e., the measured increase in micro-organism count over time without the presence of the hydrogels of the present invention. The micro-organism reduction result for each sample is listed in the following table:

| Antimicrobial EXAMPLE | Percent Nitrogen | 1 Hour CFU/ml | 2 Hours CFU/ml | 3 Hours CFU/ml | 5.5 Hours CFU/ml | 24 Hours CFU/ml |
|---|---|---|---|---|---|---|
| 5 | 1.5 | <10 | <10 | <10 | <10 | <10 |
| 6 | 0.5 | 10,000 | ≈1000 | ≈500 | ≈100 | ≈100 |
| 7 | 1.5 | <10 | <10 | <10 | <10 | <10 |
| 8 | 1.5 | ≈1000 | ≈500 | ≈100 | ≈50 | ≈10 |
| 9 | 1.3 | <10 | <10 | <10 | <10 | <10 |
| 10 | 1.7 | <10 | <10 | <10 | <10 | <10 |
| 11* | NA | 10,000 | 50,000 | 100,000 | 500,000 | 100,000 |
| NA = Not Applicable | | | | | | |

| | | | | | | |
|---|---|---|---|---|---|---|
| *Not an example of the invention; Comparative Example Only | | | | | | |

### EXAMPLE 12

The two step method described previously in Method Two and in Example 1 was used to prepare hydrogel particles by starting with EAA having a melt index of 300 grams/10 minutes and 20 percent acrylic acid content which was finely ground and sieved to a particle size of 200 mesh (75 µm). Conversion of the EAA particles to a hydrogel using didecyl dimethyl ammonium chloride as the antimicrobial yielded hydrogel powder having a nitrogen content of 2.2 percent (44.66 percent by weight active bound quaternary ammonium compound).

Portions of this powdered hydrogel were added to CLEAN & CLEAR® facial moisture lotion (made by REVLON, Inc.) at levels of 5.4 percent, 8.5 percent, 10.6 percent and 20.6 percent by weight of hydrogel. CLEAN & CLEAR contains: water, isosteryl neopentanonate, propylene glycol, TEA-stearate, glyceryl stearate, steareth-20, TEA-carbomer 941, geranium extract, dipropylene glycol, trisodium EDTA, stearic acid, methylparaben, propylparaben, imidazolidinyl urea, FD&C red #4 and FD&C yellow #5. Of these compounds, methyl paraben, propylparaben and imidazolidinyl urea are antimicrobials which are added to the formulation by the manufacturer to prevent bacterial growth during storage of the cream.

CLEAN & CLEAR lotion cream was formulated with varying levels of 200 mesh (75 µm) hydrogel particles. One weight percent of the formulated CLEAN & CLEAR was added to a portion of a test fluid. Test fluid was prepared as described in Example 1 containing approximately 1,000 CFU/ml of micro-organisms (measured using the EASICULT® system). Using the EASICULT® test procedure described in Example 3, the efficacy of each of the formulated Clean & Clear samples was determined. The following table describes test results after adding the hydrogel at the active bound quaternary ammonium compound concentration indicated:

| Sample | Active Bound Quat. Ammon. Cmpnd. Concn. *** (ppm) | CFU/ml After 1 Hour | CFU/ml After 2.5 Hours | CFU/ml After 3 Hours | CFU/ml After 5.5 Hours |
|---|---|---|---|---|---|
| Test fluid | 0 | 1000 | 5000 | 10,000 | 1,000,000 |
| A* | 0 | 1000 | 5000 | 10,000 | 1,000,000 |
| B | 250 | <10 | ≈10 | ≈100 | 100,000 |
| C | 400 | <10 | <10 | ≈10 | ≈100 |
| D | 500 | <10 | <10 | <10 | ≈10 |
| E** | 500 | <10 | <10 | <10 | ≈10 |
| F | 1000 | <10 | <10 | <10 | <10 |

| | | | | | |
|---|---|---|---|---|---|
| * Contains 1% by weight CLEAN & CLEAR without hydrogel | | | | | |
| ** Contains 500 ppm of the active bound quaternary ammonium compound, but 0% CLEAN & CLEAR | | | | | |
| ***approximate level in the test fluid | | | | | |

The data indicate that addition of hydrogel particles to CLEAN & CLEAR lotion cream was effective in destroying micro-organisms. Sample A shows that diluted CLEAN & CLEAR alone was not effective as a antimicrobial. Sample E clearly demonstrates that the hydrogel was responsible for the antimicrobial activity. Samples B, C, D and F show that different levels of hydrogel result in different levels of effectiveness and thus afford a formulator flexibility dependant upon end use.

### EXAMPLE 13

The 200 mesh (75 µm) hydrogel particles of Example 12 were added to commercial toothpastes to show effectiveness in controlling micro-organisms. These commercial toothpastes and their reported contents were as follows:

GLEEM® by Procter & Gamble: sodium fluoride in a dentifrice base of calcium pyrophosphate, water, sorbitol, glycerin, cellulose gum, sodium lauryl sulfate, flavor, magnesium aluminum silicate, disodium phosphate, sodium phosphate, and sodium saccharin; and

ULTRA BRITE by Colgate-Palmolive Company: Sodium monofluorophosphate, water, glycerin, hydrated silica, alumina, sodium lauryl sulfate, flavor, carrageenen, cellulose gum, sodium benzoate, titanium dioxide and sodium saccharin.

Portions (0.91 grams) of the 200 mesh (75 µm) hydrogel particles were mixed with 3 gram portions of two commercial toothpastes. The entire amount of each formulated mixture was added to 500 ml of test fluid prepared as in Example 1 containing approximately 100,000 CFU/ml micro-organisms to yield a mixture containing 910 ppm active bound quaternary ammonium compound and 6000 ppm toothpaste in the test fluid. Using the EASICULT® test procedure described in Example 3, the efficacy of each of the formulated toothpaste samples was determined. The results are displayed in the following table:

| Trial | Base Toothpaste | Active Bound Quaternary Ammonium Compound Approximate Concentration (ppm) | CFU/ml in 1 Hour | CFU/ml in 2 Hours | CFU/ml in 3 Hours |
|---|---|---|---|---|---|
| A | GLEEM* | 0 | 100,000 | 100,000 | 100,000 |
| B | GLEEM* | 910 | 500 | 100 | 100 |
| C | ULTRABRITE** | 0 | 100,000 | 100,000 | 100,000 |
| D | ULTRABRITE** | 910 | 500 | 100 | <10 |

| | | | | | |
|---|---|---|---|---|---|
| * Tradename of PROCTER & GAMBLE | | | | | |
| ** Tradename of COLGATE-PALMOLIVE Company | | | | | |

As the data indicate, Trials C and E, which contain the hydrogel particles of the invention, control the micro-organisms in a matter of hours, whereas comparative Trials A, B and D, which do not contain hydrogel, had no effect on the micro-organism population.

### EXAMPLE 14

Similarly, to Example 13, 0.91 gram portions of the 200 mesh (75 µm) hydrogel particles prepared in Example 12 were added to 3 gram portions of commercially available shampoos. Each formulated shampoo sample was added to an aliquot of nutrient enriched test fluid (prepared as in Example 1) containing approximately 10,000 CFU/ml micro-organisms. Antimicrobial activity was tested using the EASICULT® TTC SYSTEM as described in Example 3. The table below summarizes the data:

| Trial | Base Shampoo | Active Bound Quaternary Ammonium Compound Approximate Concentration (ppm) | CFU/ml in 2 Hours | CFU/ml in 3 Hours | CFU/ml in 4 Hours |
|---|---|---|---|---|---|
| A | Test Fluid Only | 0 | 50,000 | 100,000 | 100,000 |
| B | CLAIROL* | 0 | 50,000 | 100,000 | 100,000 |
| C | CLAIROL* | 910 | 1,000 | 100 | <10 |

| | | | | | |
|---|---|---|---|---|---|
| * Made by Clairol, Inc. | | | | | |

As the data indicate, Trial C, which contained the hydrogel particles of the invention, controlled the micro-organisms in a matter of hours, whereas comparative Trials A and B, which did not contain hydrogel, had no effect on the micro-organism population.

### EXAMPLE 15

Similarly to Example 13, 0.91 gram portions of the 200 mesh (75 µm) hydrogel particles prepared in Example 13 were added to 3 gram portions of three commercially available soaps. Each formulated mixture was added to 500 ml of test fluid prepared as in Example 1 containing approximately 100,000 CFU/ml micro-organisms to yield a mixture containing 910 ppm active bound quaternary ammonium compound and 6000 ppm soap. Using the EASICULT® test procedure described in Example 3, the efficacy of each of the formulated soap samples was determined. The results are displayed in the following table:

| Trial | Base Soap | Active Bound Quaternary Ammonium Compound Approximate Conc. (ppm) | CFU/ml in 1 Hour | CFU/ml in 2 Hours | CFU/ml in 3 Hours |
|---|---|---|---|---|---|
| A | OXYDOL* Flake | 0 | 100,000 | 75,000 | 100,000 |
| B | OXYDOL* Flake | 910 | 50,000 | 10,000 | 5,000 |
| C | IVORY** Hand | 0 | 100,000 | 100,000 | 100,000 |
| D | IVORY** Hand | 910 | 1,000 | 100 | 10 |
| E | SUN*** Liquid | 0 | 100,000 | 100,000 | 100,000 |
| F | SUN*** Liquid | 910 | 5,000 | 1000 | 10 |

| | | | | | |
|---|---|---|---|---|---|
| * Tradename of Procter & Gamble | | | | | |
| ** Tradename of Procter & Gamble | | | | | |
| *** Tradename of Lever Brothers Company | | | | | |

Trials B, D and F show that the addition of the hydrogel particles of the invention to commercially available soaps controlled micro-organisms in water. Trials A, C and E show that the commercially available soaps did not control micro-organisms by themselves.

### EXAMPLE 16

Using Method One, a hydrogel was prepared from melt spun EAA with cetyl pyridinium chloride (CPC) substituted for didecyl dimethyl ammonium chloride. The active level of the antimicrobial cation (or active bound quaternary ammonium compound) after this reaction was 2.1 percent by weight nitrogen or 48.4 percent by weight CPC. Portions of the hydrogel fiber were added to 500 ml aliquots of test fluid (prepared as in Example 1) containing approximately 100,000 CFU/ml micro-organisms to yield mixtures containing from 16 ppm active bound quaternary ammonium compound to 517 ppm active bound quaternary ammonium compound. Trial A did not contain hydrogel and was a comparative trial only. Using the EASICULT® test procedure described in Example 3, the efficacy of each of the formulated samples was determined. The results are displayed in the following table:

| Trial | Active Bound Quaternary Ammonium Compound Approximate Conc. (ppm) | CFU/ml in 1 Hour | CFU/ml in 2 Hours | CFU/ml in 3 Hours | CFU/ml in 4 Hours |
|---|---|---|---|---|---|
| A* | 0 | 100,000 | 100,000 | 100,000 | 100,000 |
| B | 16 | 50 | 10 | 10 | 10 |
| C | 30 | <10 | <10 | <10 | <10 |
| D | 62 | <10 | <10 | <10 | <10 |
| E | 86 | <10 | <10 | <10 | <10 |
| F | 172 | <10 | <10 | <10 | <10 |
| G | 258 | <10 | <10 | <10 | <10 |
| H | 346 | <10 | <10 | <10 | <10 |
| I | 430 | <10 | <10 | <10 | <10 |
| J | 517 | <10 | <10 | <10 | <10 |

| | | | | | |
|---|---|---|---|---|---|
| * Test fluid without hydrogel added | | | | | |

The data show that hydrogels made in accordance with the invention using cetyl pyridinium chloride as the antimicrobial and placed into micro-organism rich environments were effective in reducing bacteria counts, even down to a level of 16 ppm active bound quaternary ammonium compound.

### EXAMPLE 17 - For Comparison only; not an example of the invention

CEPACOL™ mouthwash (made by Lakeside Pharmaceuticals), which contains 500 ppm of cetyl pyridinium chloride (CPC), was added in various aliquots to test fluid prepared as in Example 1 which contained approximately 1,000,000 CFU/ml. The level of the CEPACOL was adjusted in each trial to vary the active level of CPC. Using the EASICULT® test procedure described in Example 3, the efficacy of each of the formulated samples was determined. The data are summarized below:

| Trial | Active CPC Content (ppm) | CFU/ml in 1 Hour | CFU/ml in 4 Hours |
|---|---|---|---|
| A* | 0 | 1,000,000 | 1,000,000 |
| B | 5 | 50,000 | NM |
| C | 10 | 5,000 | NM |
| D | 15 | 500 | NM |
| E | 20 | 100 | NM |
| F | 25 | 10 | <10 |
| G | 50 | <10 | <10 |
| H | 100 | <10 | <10 |
| I | 200 | <10 | <10 |

| | | | |
|---|---|---|---|
| * Test fluid only without CEPACOL added NM indicates data Not Measured | | | |

As the data indicate, CPC was effective as an antimicrobial to low levels. Control of micro-organisms was exhibited within one hour of contact when the CPC level was as low as 25 ppm. As Example 16 demonstrated in comparison to Example 17, when CPC was utilized as the active antimicrobial of the present invention in hydrogel form, the hydrogels were capable of performing as antimicrobials at approximately the same active CPC level as a commercially available mouthwash.

### EXAMPLES 18-19 and COMPARATIVE EXAMPLE 20

Antimicrobial hydrogels were prepared from EAA cast film and didecyl dimethyl ammonium chloride (Example 18), CPC (Example 19) and 8-hydroxy quinoline (Comparative Example 20) using the one-step process previously described. The hydrogels were tested for activity using the zone of inhibition test described in Example 2 and test fluid prepared as described in Example 1 with initial plate count at >10,000 CFU/ml micro-organisms. The samples were also tested for percent nitrogen, indicating the presence or absence and level of the specific antimicrobial. Each hydrogel film sample was tested at the beginning of the procedure (i.e., before contact with water) and after 7 days of daily 24 hour stagnant soakings in deionized (DI) water (pH=4.52). Each of the samples was individually placed in 125 ml (approximately 4 US ounce) aliquots of fresh DI water.
The test results are described in the following tables:

| Example | Antimicrobial | Starting Percent Nitrogen | Starting Weight Percent Bound Quat. | Remaining Percent Nitrogen | Remaining Weight Percent Bound Quat. |
|---|---|---|---|---|---|
| 18 | didecyl dimethyl ammonium chloride | 0.86 | 20.03 | 0.86 | 20.02 |
| 19 | CPC | 0.57 | 12.4 | 0.54 | 11.75 |
| 20* | 8-hydroxy quinoline | 0.09 | 0.35 | 0.005 | 0.02 |

| | | | | | |
|---|---|---|---|---|---|
| * Comparative example only | | | | | |

| Example | Antimicrobial | Beginning Zone of Inhibition (mm) | Zone of Inhibition After 7 Days DI Water Contact |
|---|---|---|---|
| 18 | didecyl dimethyl ammonium chloride | NGCA** | NGCA** |
| 19 | CPC | NGCA** | NGCA** |
| 20* | 8-hydroxy quinoline | 5.5 | None*** |

| | | | |
|---|---|---|---|
| *Comparative example only | | | |
| **No Growth on Contact Area | | | |
| ***No zone of inhibition (i.e., growth everywhere, including contact area) | | | |

As the data indicate, Examples 18 and 19 did not have a measurable zone of inhibition, although they did not have growth detected beneath the sample, and did retain the antimicrobial, as measured by percent nitrogen, at levels from 94-100 percent (by weight of the original level) after 7 days of contact with DI water. Thus, the antimicrobials for use with the present invention were essentially non-leachable when used in manufacturing the hydrogels of the present invention. As previous Examples using the EASICULT® system demonstrated, each of these hydrogels controled micro-organisms on contact without having a measurable zone of inhibition.

In contrast, when 8-hydroxy quinoline was used as the antimicrobial, the zone of inhibition was initially high, indicating antimicrobial migration, and was reduced after 7 days of contact with DI water to no detectable zone of inhibition, with growth detected under the sample. The percent nitrogen analysis verified the rapid depletion or migration of 8-hydroxy quinoline from the hydrogel structure, thus showing its leachability.

### Example 21

A dispersion of antimicrobial hydrogels was formed by the following procedure:
1. An amount of a quaternary ammonium salt having antimicrobial properties was added to an amount of deionized water and stirred. The pH was adjusted to the indicated target, thus forming a first solution.
2. An ethylene/acrylic acid dispersed solution (dispersed using any of the methods known to those skilled in the art, for example, by mixing with an aqueous dispersion of ammonium hydroxide) was diluted with deionized water to a predetermined degree and also stirred to form a second solution.
3. The entire second solution was slowly added to the first solution while simultaneously stirring to prevent solids aggregation, thus forming a third solution.
4. The pH of the third solution was adjusted to the desired point until the pH was relatively constant, thus forming the antimicrobial hydrogel dispersion.

Percent non-dispersibles was determined by filtering an aliquot of the dispersion through a 100 mesh (150 micro-meters) screen and weighing the amount of the dispersion collected on the screen. The percent solids was determined by calculating the amount of the solids originally added to the solution minus the amount of the non-dispersibles. The following table describes the quantities used in dispersions formed from the antimicrobial hydrogels of the present invention:

| Trial | First Solution | | Second Solution | | Percent Conversion |
|---|---|---|---|---|---|
| | Weight of Quat. Amm. Salt added (grams) | Weight of water added (grams) | Weight of EAA dispersion added (grams) | Weight of water added (grams) | |
| 1A-D | 10 | 100 | 20 | 70 | 100 |
| 2A-D | 7.5 | 100 | 20 | 70 | 75 |
| 3A-D | 5 | 100 | 20 | 70 | 50 |

The following three tables list the percent non-disperibles and percent solids for these three experiments as a function of pH:

| Trial | Target pH | Weight percent solids | Weight percent non-dispersibles |
|---|---|---|---|
| 1A | 4 | 4.70 | 62.22 |
| 1B | 6 | 5.47 | 96.01 |
| 1C | 8 | 7.68 | 98.32 |
| 1D | 10 | 3.65 | 2.09 |
| Target conversion = 50 percent | | | |

| Trial | Target pH | Weight percent solids | Weight percent non-dispersibles |
|---|---|---|---|
| 2A | 4 | 5.36 | 65.03 |
| 2B | 6 | 5.61 | 76.75 |
| 2C | 8 | 7.23 | 98.49 |
| 2D | 10 | 6.75 | 93.87 |
| Target conversion = 75 percent | | | |

| Trial | Target pH | Weight percent solids | Weight percent non-dispersibles |
|---|---|---|---|
| 3A | 4 | 5.13 | 7.88 |
| 3B | 6 | 4.99 | 1.05 |
| 3C | 8 | 4.93 | 76.74 |
| 3D | 10 | 3.36 | 90.89 |
| Target conversion = 100 percent | | | |

It is important to note that one or more surfactants can be added to the dispersion, but it is not necessary to add surfactant for the dispersion to be formed.

The efficacy of dispersion trial 3B was demonstrated by performing a zone of inhibition test, as described in Example 2, using the POLYSEED® (made by POLYBAC) Bacterial Inoculum test solution described in Example 22. The dispersion was added dropwise to each of the indicated substrates and subsequently air dried overnight. The dispersion coated substrate was weighed and reweighed to determine the amount of dispersion added. The following table describes the test results:

| Substrate | Dispersion amount applied to substrate (grams) | Zone of Inhibition (mm) |
|---|---|---|
| NA | NA | 3.25 |
| nylon scrim | 0.002 | 0.75 |
| filter paper | 0.0103 | 1.0 |
| NA = Not Applicable; this sample is the antimicrobial hydrogel which is cold pressed into a plaque | | |

As the data indicate, the zone of inhibition was minimal for all the samples, but the antimicrobial hydrogel dispersions still maintained efficacy.

### Example 22

Blends of hammer milled hydrogel fiber having approximately 1.9 percent nitrogen prepared as in Example 1 and other thermoplastic polymers (low density polyethylene (LDPE), ethylene-carbon monoxide copolymers (ECO), and ethylene-acrylic acid interpolymers (EAA)) were prepared by preweighing each sample and adding to a modified Banbury mixer for melt mixing. The antimicrobial hydrogels were allowed to air dry before the experiment was performed. The diluent thermoplastic was added to the Banbury first and melted. Subsequently, the antimicrobial hydrogel was added to the Banbury and allowed to blend for 2 minutes. For a polyethylene based diluent thermoplastic, the temperature was approximately 140°C. For a 100 percent antimicrobial hydrogel melt blended sample, the melt temperature is approximately 130°C. After the 2 minute mixing period, the polymer/antimicribial hydrogel blend was pressed into 5 cm (2 inch) diameter disks, each 0.3 cm (0.125 inch) thick in a hot press at 190°C for 2 minutes. The pressed disks were subsequently tested for antimicrobial activity using the zone of inhibition testing previously described in Example 2 using a POLYSEED® Bacterial Inoculum test solution prepared according to the following recipe:

POLYSEED® is a blend of twelve types of bacteria (rods and cocci) commonly found in municipal and industrial wastes which has been formulated as a seed population for biochemical oxygen demand (BOD) tests. In practice it has been found that bacterial populations on the order of 10⁶ colony forming units (CFU) per ml can be expected with one POLYSEED® capsule. This population is stable for up to six hours with stirring and aeration.
1. Reagent Preparation
   Prepare aqueous reagent solutions as follows:
   a. Magnesium sulfate solution: Dissolve 3.35 g MgSO₄·7H₂O in distilled water and dilute to one hundred mls.
   b. Calcium chloride solution: Dissolve 3.75 g CaCl₂ in distilled water and dilute to one hundred mls.
   c. Ferric chloride solution: Dissolve 0.025 g FeCl₃·6H2O in distilled water and dilute to one hundred mls.
   d. Phosphate buffer solution: Dissolve .85 g KH₂PO₄, 2.175 g K₂HPO₄, 3.34 g Na₂HPO₄·7H₂O, and .17 g NH₄Cl in about 50ml of distilled water and dilute to 100 ml. The pH should be 7.2 without further adjustment.
   e. Place prepared solutions in bottles for storage.
   NOTE: Discard any of the above reagents if there is any sign of biological growth in the stock solutions.
2. Measure 500 ml of distilled water into a one liter beaker.

3. Add one ml of each of the prepared reagents (see step 1) to the distilled water and stir to mix.
4. Add the contents of one POLYSEED® capsule to the water.
5. Place a magnetic stirring bar into the solution and stir on a stir plate and aerate with a sparge tube for at least one hour.
6. After one hour, remove an aliquot of the POLYSEED® solution.
7. Expose an EASICULT-TTC slide to this solution and incubate as per the instructions for its use.
8. Continue to stir and aerate the POLYSEED® solution as aliquots are withdrawn for various purposes. The solution is good for at least six (6) hours after preparation.
9. At the conclusion of testing or at the end of the day, dispose of the solution.

The following tables describe the melt blended antimicrobial hydrogel/thermoplastic polymer blends and the resultant antimicrobial activity:

| Weight Percent Hydrogel | Diluent Polymer | Zone of Inhibition (mm) | Antimicrobial Efficacy* |
|---|---|---|---|
| 50 | EAA (3 percent acrylic acid) | 2.0 | NGOC |
| 50 | ECO (9.8 percent carbon monoxide) | 3.0 | NGOC |
| 100 | None | 4.65 | NGOC |
| 100** | None | 3.75 | NGOC |

| | | | |
|---|---|---|---|
| *NGOC = No Growth On the Contact area | | | |
| ** Not Melt blended | | | |

| Diluent polymer = EAA (melt index = 1.5 g/10 minutes, 10 percent acrylic acid) | | | |
|---|---|---|---|
| Weight Percent Hydrogel | Melt index (g/10 minutes) | Zone of Inhibition (mm) | Antimicrobial Efficacy* |
| 50 | 29 | 0 | TGOC |
| 25 | 3.9 | 0 | TGOC |
| 12.5 | 2.5 | 0 | GOC |
| 6.25 | 2.2 | 0 | GOC |
| 3.125 | 1.8 | 0 | GOC |
| 0 | 1.5 | 0 | GOC |

| | | | |
|---|---|---|---|
| *TGOC = Trace growth on the Contact area GOC = Growth on the Contact area | | | |

| Diluent polymer = LDPE (melt index = 2 g/10 minutes, density = 0.9225 g/cc) | | | |
|---|---|---|---|
| Weight Percent Hydrogel | Melt index (g/10 minutes) | Zone of Inhibition (mm) | Antimicrobial Efficacy* |
| 50 | 143 | 2.25 | NGOC |
| 25 | 18 | 0.75 | NGOC |
| 12.5 | 5 | 0.5 | NGOC |
| 6.25 | 3.4 | 0 | TGOC |
| 3.125 | 2.9 | 0 | GOC |
| 0 | 2 | 0 | GOC |

| | | | |
|---|---|---|---|
| *NGOC = No Growth On the Contact area TGOC = Trace Growth on the Contact area GOC = Growth on the Contact area | | | |

As the data in the tables demonstrate, melt blends of antimicrobial hydrogels of the present invention with other thermoplastics, especially LDPE, ECO, and EAA having relatively low acrylic acid content, maintained effectiveness as antimicrobials, with minimal zones of inhibition, but without growth on the contact area.

## Claims

1. A water-swollen but water-insoluble antimicrobial hydrogel obtainable by reacting at least one vinyl addition shaped interpolymer having pendant alkali metal or ammonium carboxylate groups with at least one antimicrobial compound selected from quaternary ammonium salts, protonated tertiary ammonium salts of Formula II herein, and chlorhexidine, thereby ionically bonding said antimicrobial compound to the pendant carboxylate groups and rendering the antimicrobial compound essentially non-leachable from the hydrogel when contacted with deionized water.

2. A hydrogel as claimed in Claim 1, wherein at least 75% of the pendant carboxylate groups are ionically bonded to said antimicrobial compound.

3. A hydrogel as claimed in Claim 1 or Claim 2, wherein said antimicrobial compound is chlorhexidine.

4. A hydrogel as claimed in Claim 1 or Claim 2, wherein said antimicrobial compound is a quaternary ammonium salt.

5. A hydrogel as claimed in Claim 1 or Claim 2, wherein said antimicrobial compound has a molecular structure corresponding to the formula: 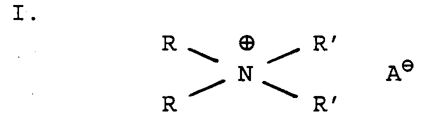 wherein
each R' independently is -CH₃ or -C₂H₅ and each R independently is C₂H₅-C₂₀H₄₁
A^{⊖} = compatible neutralizing inert anion, 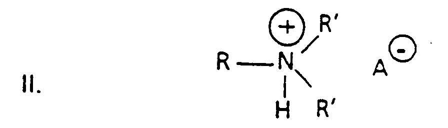 wherein
each R' is independently **-**CH₃ or -C₂H_{**5**} and
R = C₂H₅-C₂₀H₄₁
A^{⊖} = compatable neutralizing inert anion. or 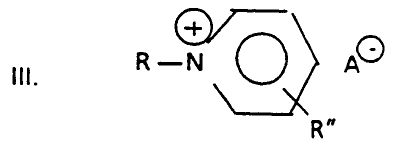 wherein
R = C₂H₅-C₂₀H₄₁
R'' = inert substituent or -H
A^{⊖} = compatable neutralizing inert anion.

6. A hydrogel as claimed in Claim 5, wherein said antimicrobial compound is didecyl dimethyl ammonium chloride or bromide, cetyl pyridinium chloride or bromide, dimethyl benzyl ammonium chloride or bromide, or a mixture of any of these.

7. A hydrogel as claimed in any one of the preceding claims, wherein said vinyl addition interpolymer is an ionomer of an interpolymer of ethylene with an alpha-olefinically unsaturated carboxylic acid or ester.

8. A hydrogel as claimed in Claim 7, wherein said vinyl addition interpolymer is an ionomer of an ethylene-acrylic acid interpolymer.

9. A hydrogel as claimed in Claim 8, wherein said vinyl addition interpolymer is a sodium ionomer of an ethylene-acrylic acid interpolymer.

10. A hydrogel as claimed in any one of the preceding claims, wherein said hydrogel is substantially dehydrated.

11. A hydrogel as claimed in Claim 10, wherein said hydrogel is water absorbent or water-swellable.

12. A hydrogel as claimed in any one of the preceding claims, wherein pendant alkali metal carboxylate or ammonium carboxylate groups have been ion-exchanged with at least one mono-, di-, or tri-valent metal cation to elevate the melting point of the hydrogel.

13. An antimicrobial composition comprising a hydrogel as claimed in any one of the preceding claims, blended with one or more thermoplastics and melt processed into a desired shape.

14. A composition as claimed in Claim 13, wherein said thermoplastic is chosen from polypropylene, low density polyethylene, linear low density polyethylene and ethylene-acrylic acid interpolymers.

15. A method of making a water-swollen but water-insoluble antimicrobial hydrogel as defined in Claim 1 comprising
(A) reacting, in aqueous reaction medium,
(a) at least one shaped vinyl addition interpolymer having pendant alkali metal carboxylate or ammonium carboxylate groups with
(b) at least one antimicrobial compound selected from quaternary ammonium salts, protonated tertiary ammonium salts of Formula II herein, and chlorhexidine and
(B) recovering the reaction product as said antimicrobial hydrogel.

16. A method as claimed in Claim 15, wherein the vinyl addition interpolymer ionomer is formed in situ in a one step process in which the shaped parent interpolymer is simultaneously contacted with aqueous base of at least pH 7.1 and said antimicrobial compound.

17. A method as claimed in Claim 15, wherein the vinyl addition interpolymer ionomer is formed in a first step by contacting the shaped parent interpolymer with aqueous base of at least pH 7.1 and said ionomer is contacted in a second step with said antimicrobial compound in a dilute aqueous solution of pH 6 to pH 7.

18. A method as claimed in Claim 16 or Claim 17, wherein the parent interpolymer is in the form of a stretched fiber or strand.

19. A method of making a water-swollen but water-insoluble antimicrobial hydrogel as defined in Claim 1 comprising melt mixing at least one shaped vinyl addition interpolymer with at least one antimicrobial compound selected from quaternary ammonium salts, protonated tertiary ammonium salts of Formula II herein, and chlorhexidine while simultaneously or incrementally adding an aqueous caustic solution.

20. A method as claimed in any one of Claims 15 to 19, wherein the vinyl addition interpolymer ionomer and/or antimicrobial compound is as defined in any one of Claims 3 to 9.

21. A method as claimed in any one of Claims 15 to 20, further comprising the step of substantially dehydrating said hydrogel.

22. A method as claimed in any one of Claims 15 to 20, further comprising ion exchanging said pendant alkali metal carboxylate or ammonium carboxylate groups with at least one mono-, di-, and/or tri-valent metal cation to form an antimicrobial hydrogel having an elevated melting point.

23. The use of an antimicrobial hydrogel as defined in Claim 1 to control micro-organisms.

24. A use as claimed in Claim 23, wherein the hydrogel is as defined in any one of Claims 2 to 12.

25. A method of removing water from an aqueous fluid comprising contacting said aqueous fluid with the water absorbent hydrogel of Claim 11.

## Patentansprüche

1. Wassergequollenes aber wasserunlösliches antimikrobielles Hydrogel, erhältlich durch Umsetzen von mindestens einem durch Vinyladdition gebildeten Interpolymer das Alkalimetall- oder Ammoniumcarboxylatseitengruppen aufweist, mit mindestens einer antimikrobiellen Verbindung, ausgewählt aus quartären Ammoniumsalzen, protonierten tertiären Ammoniumsalzen der hierin angegebenen Formel II, und Chlorhexidin, wobei die antimikrobielle Verbindung an die Carboxylatseitengruppen ionisch gebunden wird und die antimikrobielle Verbindung im wesentlichen aus dem Hydrogel nicht auslaugbar gemacht wird, wenn es mit entionisiertem Wasser kontaktiert wird.

2. Hydrogel nach Anspruch 1, worin mindestens 75 % der Carboxylatseitengruppen ionisch an die antimikrobielle Verbindung gebunden sind.

3. Hydrogel nach Anspruch 1 oder Anspruch 2, worin die antimikrobielle Verbindung Chlorhexidin ist.

4. Hydrogel nach Anspruch 1 oder Anspruch 2, worin die antimikrobielle Verbindung ein quartäres Ammoniumsalz ist.

5. Hydrogel nach Anspruch 1 oder Anspruch 2, worin die antmikrobielle Verbindung eine Molekülstruktur entsprechend der Formel: 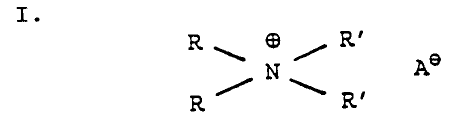 aufweist, worin jedes R' unabhängig -CH₃ oder -C₂H₅ ist und jedes R unabhängig C₂H₅-C₂₀H₄₁ ist,
A⁻ = kompatibles, neutralisierendes, inertes Anion, 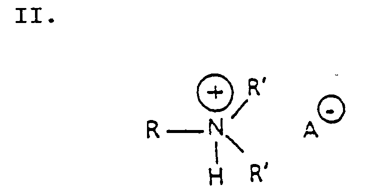 worin jedes R' unabhängig CH₃ oder -C₂H₅ ist und
R = C₂H₅-C₂₀H₄₁
A⁻ = kompatables, neutralisierendes, inertes Anion,
oder 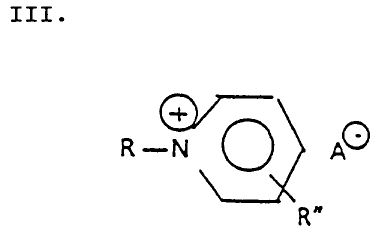 worin R = C₂H₅-C₂₀H₄₁
R'' = inerter Substituent oder -H
A⁻ = kompatables, neutalisierrendes, inertes Anion.

6. Hydrogel nach Anspruch 5, worin die antimikrobielle Verbindung Didecyldimethylammoniumchlorid oder -bromid, Cetylpyridiniumchlorid oder -bromid, Dimethylbenzylammoniumchlorid oder -bromid, oder ein Gemisch von beliebigen aus diesen ist.

7. Hydrogel nach einem der vorhergehenden Ansprüche, worin das Vinyladditionsinterpolymer ein Ionomer eines Interpolymers von Ethylen mit einer alpha-olefinisch ungesättigten Carbonsäure oder einem alpha-olefinisch ungesättigten Ester ist.

8. Hydrogel nach Anspruch 7, worin das Vinyladditionsinterpolymer ein Ionomer eines Ethylen-Acrylsäure-Interpolymers ist.

9. Hydrogel nach Anspruch 8, worin das Vinyladditionsinterpolymer ein Natriumionomer eines Ethylen-Acrylsäure-Interpolymers ist.

10. Hydrogel nach einem der vorhergehenden Ansprüche, worin das Hydrogel im wesentlichen dehydratisiert ist.

11. Hydrogel nach Anspruch 10, worin das Hydrogel wasserabsorbierend oder wasserquellbar ist.

12. Hydrogel nach einem der vorhergehenden Ansprüche, worin Alkalimetallcarboxylat- oder Ammoniumcarboxylatseitengruppen mit mindestens einem mono-, di- oder trivalenten Metallkation ionenausgetauscht worden sind, um den Schmelzpunkt des Hydrogels zu erhöhen.

13. Antimikrobielle Zusammensetzung, umfassend ein Hydrogel nach einem der vorhergehenden Ansprüche, gemischt mit einem oder mehreren Thermoplasten und in eine gewünschte Form schmelzverarbeitet.

14. Zusammensetzung nach Anspruch 13, worin der Thermoplast ausgewählt ist aus Polypropylen, Polyethylen mit niederer Dichte, linearem Polyethylen mit niederer Dichte und Ethylen-Acrylsäure-Interpolymeren.

15. Verfahren zum Herstellen eines wassergequollenen aber wasserunlöslichen antimikrobiellen Hydrogels nach Anspruch 1, umfassend
(A) Umsetzen in wässrigem Reaktionsmedium von
(a) mindestens einem geformten Vinyladditionsinterpolymer mit Alkalimetallcarboxylat- oder Ammoniumcarboxylatseitengruppen mit
(b) mindestens einer antimikrobiellen Verbindung, ausgewählt aus quartären Ammoniumsalzen, protonierten tertiären Ammoniumsalzen der hierin angegebenen Formel II und Chlorhexidin, und
(B) Gewinnen des Reaktionsprodukts als das antimikrobielle Hydrogel.

16. Verfahren nach Anspruch 15, worin das Vinyladditionsinterpolymerionomer in situ in einem Einstufenverfahren gebildet wird, in welchem das geformte Ausgangsinterpolymer gleichzeitig mit wässriger Base mit einem pH von mindestens 7,1 und der antimikrobiellen Verbindung kontaktiert wird.

17. Verfahren nach Anspruch 15, worin das Vinyladditionsinterpolymerionomer in einem ersten Schritt durch Kontaktieren des geformten Ausgangsinterpolymers mit wässriger Base mit einem pH von mindestens 7,1 gebildet wird und das Ionomer in einem zweiten Schritt mit der antimikrobiellen Verbindung in einer verdünnten wässrigen Lösung mit pH 6 bis pH 7 kontaktiert wird.

18. Verfahren nach Anspruch 16 oder 17, worin das Ausgangsinterpolymer in der Form einer gestreckten Faser oder eines gestreckten Stranges vorliegt.

19. Verfahren zum Herstellen eines wassergequollenen aber wasserunlöslichen antimikrobiellen Hydrogels nach Anspruch 1, umfassend Schmelzmischen von mindestens einem geformten Vinyladditionsinterpolymer mit mindestens einer antimikrobiellen Verbindung, ausgewählt aus quartären Ammoniumsalzen, protonierten tertiären Ammoniumsalzen der hierin angegebenen Formel II und Chlorhexidin, wobei gleichzeitig oder schrittweise eine wässrige Alkalilösung zugegeben wird.

20. Verfahren nach einem der Ansprüche 15 bis 19, worin das Vinyladditionsinterpolymerionomer und/oder die antimikrobielle Verbindung wie in einem der Ansprüche 3 bis 9 definiert ist.

21. Verfahren nach einem der Ansprüche 15 bis 20, weiterhin umfassend den Schritt des im wesentlichen Dehydratisierens des Hydrogels.

22. Verfahren nach einem der Ansprüche 15 bis 20, weiterhin umfassend Ionenaustauschen der Alkalimetallcarboxylat- oder Ammoniumcarboxylatseitengruppen durch mindestens ein mono-, di- und/oder trivalentes Metallkation, um ein antimikrobielles Hydrogel mit einem erhöhten Schmelzpunkt zu bilden.

23. Verwendung eines antimikrobiellen Hydrogels nach Anspruch 1, um Mikroorganismen zu bekämpfen.

24. Verwendung nach Anspruch 23, worin das Hydrogel wie in einem der Ansprüche 2 bis 12 definiert ist.

25. Verfahren zum Entfernen von einem wässrigen Fluid, umfassend das Kontaktieren des wässrigen Fluids mit dem wasserabsorbierenden Hydrogel nach Anspruch 11.

## Revendications

1. Hydrogel qui peut gonfler dans l'eau, mais ne peut pas s'y dissoudre, et qu'on peut obtenir en faisant réagir au moins un interpolymère vinylique d'addition, mis sous une certaine forme et comportant des groupes latéraux de type carboxylate de métal alcalin ou d'ammonium, avec au moins un composé antimicrobien choisi parmi les sels d'ammonium quaternaire, les sels d'ammonium tertiaire protoné de formule II indiquée plus loin et la chlorhexidine, ce qui entraîne l'établissement de liaisons ioniques entre ledit composé antimicrobien et les groupes carboxylate latéraux et rend ainsi le composé antimicrobien pratiquement impossible à extraire de l'hydrogel quand celui-ci est mis en contact avec de l'eau désionisée.

2. Hydrogel conforme à la revendication 1, dans lequel au moins 75 % des groupes carboxylates latéraux sont liés par liaison ionique audit composé antimicrobien.

3. Hydrogel conforme à la revendication 1 ou 2, dans lequel ledit composé antimicrobien est de la chlorhexidine.

4. Hydrogel conforme à la revendication 1 ou 2, dans lequel ledit composé antimicrobien est un sel d'ammonium quaternaire.

5. Hydrogel conforme à la revendication 1 ou 2, dans lequel ledit composé antimicrobien possède une structure moléculaire correspondant à la formule 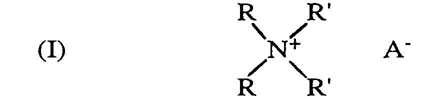 dans laquelle chaque R' représente indépendamment un groupe -CH₃ ou -C₂H₅, chaque R représente indépendamment un groupe de -C₂H₅ à -C₂₀H₄₁, et A⁻ représente un anion inerte compatible de neutralisation, 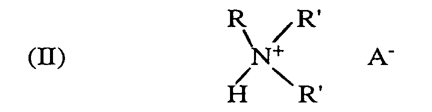 dans laquelle chaque R' représente indépendamment un groupe -CH₃ ou -C₂H₅, R représente un groupe de -C₂H₅ à -C₂₀H₄₁, et A⁻ représente un anion inerte compatible de neutralisation,
ou 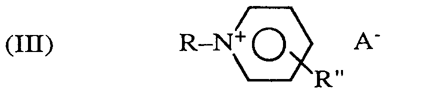 dans laquelle R représente un groupe de -C₂H₅ à -C₂₀H₄₁, R'' représente un atome d'hydrogène ou un substituant inerte et A⁻ représente un anion inerte compatible de neutralisation.

6. Hydrogel conforme à la revendication 5, dans lequel ledit composé antimicrobien est du chlorure ou bromure de didécyl-diméthyl-ammonium, du chlorure ou bromure de cétyl-pyridinium, du chlorure ou bromure de diméthyl-benzyl-ammonium, ou un mélange de n'importe lesquels de ces composés.

7. Hydrogel conforme à l'une des revendications précédentes, dans lequel ledit interpolymère vinylique d'addition est un ionomère d'un interpolymère d'éthylène et d'un ester ou acide carboxylique à insaturation α-éthylénique.

8. Hydrogel conforme à la revendication 7, dans lequel ledit interpolymère vinylique d'addition est un ionomère d'un interpolymère d'éthylène et d'acide acrylique.

9. Hydrogel conforme à la revendication 8, dans lequel ledit interpolymère vinylique d'addition est un ionomère sodique d'un interpolymère d'éthylène et d'acide acrylique.

10. Hydrogel conforme à l'une des revendications précédentes, qui est pratiquement déshydraté.

11. Hydrogel conforme à la revendication 10, qui peut absorber de l'eau ou gonfler dans l'eau.

12. Hydrogel conforme à l'une des revendications précédentes, dans lequel on a soumis les groupes latéraux de type carboxylate d'ammonium ou de métal alcalin à un échange d'ions avec au moins un cation métallique monovalent, divalent ou trivalent, afin d'élever le point de fusion de l'hydrogel.

13. Composition antimicrobienne comprenant un hydrogel conforme à l'une des revendications précédentes, mélangé avec une ou plusieurs matières thermoplastiques et mis sous la forme souhaitée par traitement à l'état fondu.

14. Composition conforme à la revendication 13, dans laquelle ladite matière thermoplastique est choisie parmi le polypropylène, le polyéthylène basse densité, le polyéthylène basse densité linéaire et les interpolymères d'éthylène et d'acide acrylique.

15. Procédé de préparation d'un hydrogel antimicrobien qui peut gonfler dans l'eau, mais ne peut pas s'y dissoudre, conforme à la revendication 1, lequel procédé comporte
A) le fait de faire réagir, en milieu réactionnel aqueux,
a) au moins un interpolymère vinylique d'addition, mis sous une certaine forme et comportant des groupes latéraux de type carboxylate de métal alcalin ou d'ammonium, avec
b) au moins un composé antimicrobien choisi parmi les sels d'ammonium quaternaire, les sels d'ammonium tertiaire protoné de formule II indiquée plus haut et la chlorhexidine, et
B) le fait de récupérer le produit de cette réaction, qui est ledit hydrogel antimicrobien.

16. Procédé conforme à la revendication 15, dans lequel on forme *in situ* un ionomère d'interpolymère vinylique d'addition, en une seule étape de procédé au cours de laquelle l'interpolymère parent, mis sous une certaine forme, est mis simultanément en contact avec ledit composé antimicrobien et avec une solution aqueuse de base dont le pH vaut au moins 7,1.

17. Procédé conforme à la revendication 15, dans lequel on forme un ionomère d'interpolymère vinylique d'addition, au cours d'une première étape, en mettant l'interpolymère parent, mis sous une certaine forme, en contact avec une solution aqueuse de base dont le pH vaut au moins 7,1, puis on met ledit ionomère, au cours d'une seconde étape, en contact avec ledit composé antimicrobien, dans une solution aqueuse diluée dont le pH vaut de 6 à 7.

18. Procédé conforme à la revendication 16 ou 17, dans lequel l'interpolymère parent se présente sous forme de fibres ou de brins étirés.

19. Procédé de préparation d'un hydrogel antimicrobien qui peut gonfler dans l'eau, mais ne peut pas s'y dissoudre, conforme à la revendication 1, lequel procédé comporte le fait de mélanger, à l'état fondu, au moins un interpolymère vinylique d'addition, mis sous une certaine forme, avec au moins un composé antimicrobien choisi parmi les sels d'ammonium quaternaire, les sels d'ammonium tertiaire protoné de formule II indiquée plus haut et la chlorhexidine, tout en y ajoutant une solution aqueuse de base caustique, tout d'un coup ou par portions.

20. Procédé conforme à l'une des revendications 15 à 19, dans lequel le ionomère d'interpolymère vinylique d'addition et/ou le composé antimicrobien sont tels qu'on les a définis dans n'importe laquelle des revendications 3 à 9.

21. Procédé conforme à l'une des revendications 15 à 20, qui comporte en outre une étape consistant à pratiquement déshydrater ledit hydrogel.

22. Procédé conforme à l'une des revendications 15 à 20, qui comporte en outre le fait de soumettre lesdits groupes latéraux de type carboxylate d'ammonium ou de métal alcalin à un échange d'ions avec au moins un cation métallique monovalent, divalent et/ou trivalent, pour obtenir un hydrogel antimicrobien à point de fusion élevé.

23. Emploi d'un hydrogel antimicrobien conforme à la revendication 1, pour lutter contre des micro-organismes.

24. Emploi, conforme à la revendication 23, d'un hydrogel conforme à l'une des revendications 2 à 12.

25. Procédé permettant d'extraire de l'eau d'un fluide aqueux, qui comporte le fait de mettre ce fluide aqueux en contact avec un hydrogel hydroabsorbant conforme à la revendication 11.
